# EUROPÄISCHE PATENTANMELDUNG

(11) **EP 1 063 233 A1**
(43) Veröffentlichungstag der Anmeldung: **27.12.2000**
(21) Anmeldenummer: 00112882.6
(22) Anmeldetag: 19.06.2000
(51) Int. Cl.: C07D 215/36

(54) **Verfahren zur Herstellung von 2-Chinolinylthioessigsäure bzw. deren Hydrochlorid**

(30) Priorität: 23.06.1999 AT 110299
(71) Anmelder: LOBA FEINCHEMIE AKTIENGESELLSCHAFT, A-2401 Fischamend (AT)
(72) Erfinder: Koller, Herbert, Dr., 1100 Wien (AT)
(74) Vertreter: Landgraf, Elvira, Dipl.-Ing.

(57) **Zusammenfassung**

Die Erfindung betrifft ein einstufiges Verfahren zur Herstellung von 2-Chinolinylthioessigsäure durch Umsetzen von 2-Chlorchinolin mit Mercaptoessigsäure.

## Beschreibung

Die Erfindung betrifft ein einstufiges Verfahren zur Herstellung von 2-Chinolinylthioessigsäure.

2-Chinolinylthioessigsäure bzw. deren Hydrochlorid ist ein bedeutendes Zwischenprodukt zur Herstellung von Stabilisatoren in photothermographischem Material, wie sie beispielsweise in der EP - A 0 631 176 beschrieben wird. 2-Chinolinylthioessigsäure wird dabei beispielsweise zu 2-Tribrommethylsulfonylchinolin umgesetzt.

Aus der Literatur sind zahlreiche Verfahren zur Herstellung von 2-Chinolinylthioessigsäure bekannt.

So ist beispielsweise aus G. F. Duffin, J. D. Kendall, J.Chem.Soc., 1951 S. 734 ff die Herstellung von 2-Chinolinylthioessigsäure aus Chinolin-2-thiol durch Umsetzung mit Chloressigsäure bekannt, wobei im vorgelagerten Verfahrensschritt 2- Chlorchinolin mit Thioharnstoff zum Chinolin-2-thiol umgesetzt wird.

Überraschenderweise konnte gefunden werden, dass 2-Chinolinylthioessigsäure in einem einstufigen Verfahren aus 2- Chlorchinolin durch direkte Umsetzung mit Mercaptoessigsäure in hoher Ausbeute und Reinheit hergestellt werden kann.

Gegenstand der Erfindung ist daher ein Verfahren zur Herstellung von 2-Chinolinylthioessigsäure der Formel I 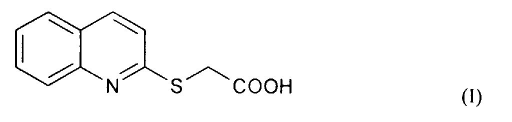 und deren Salze, dadurch gekennzeichnet, dass man 2-Chlorchinolin der Formel II 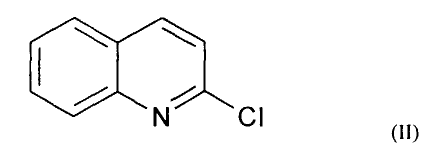 in wässriger Lösung mit Mercaptoessigsäure umsetzt und gegebenenfalls das erhaltene Salz in die freie Säure oder die freie Säure und ein Salz überführt.

Das erfindungsgemäße Verfahren verläuft mit guter Ausbeute und liefert das Produkt in für die Weiterverarbeitung ausreichender Reinheit.

Zur Durchführung des Verfahrens wird 2-Chlorchinolin aufgeschmolzen und anschließend in wässrigem Medium mit Mercaptoessigsäure versetzt. Das Reaktionsgemisch wird erhitzt, anschließend abgekühlt und die Zielverbindung I ausgefällt.

Die Umsetzung kann in basischem oder saurem wässrigem Medium erfolgen.

2-Chlorchinolin wird vorerst vorzugsweise bei einer Temperatur von 60 - 70°C aufgeschmolzen.

Erfolgt die Umsetzung in basischem Medium wird eine anorganische Base, beispielsweise wässrige NaOH, KOH u. dgl. als Säurefänger und gegebenenfalls Tetrabutylammoniumiodid als Phasentransferkatalysator zugegeben. Anschließend wird die Mercaptoessigsäure, gegebenenfalls ebenfalls als wässrige basische Lösung, zugegeben. Das Reaktionsgemisch wird auf Rückflußtemperatur erhitzt. Nach Beendigung der Reaktion wird das Reaktionsgemisch auf etwa Raumtemperatur abgekühlt und anschließend gegebenenfalls mit einem inerten organischen Lösungsmittel, beispielsweise mit einem Ether, wie t-Butylmethylether, oder Ethylacetat oder Toluol oder Benzol u. dgl. gewaschen. Die wässrige Phase wird daraufhin durch Zugabe einer anorganischen Säure, beispielsweise HCl angesäuert, wodurch das Produkt als Hydrochlorid ausgefällt wird. Die Fällung erfolgt im sauren Bereich, bei einem pH-Wert < 1. Der entstandene Niederschlag wird gewaschen, beispielsweise mit destilliertem Wasser, und anschließend getrocknet.

Die Umsetzung kann aber auch bei niedrigeren Temperaturen erfolgen. Dazu wird die aufgeschmolzene Ausgangsverbindung 2-Chlorchinolin in einem wässrigen Medium, beispielsweise in destilliertem Wasser, aufgenommen und die Mercaptoessigsäure bei einer Temperatur von 35 - 65° C , vorzugsweise 45 - 50°C zudosiert. Nach Beendigung der Reaktion wird das Reaktionsgemisch etwas abgekühlt, vorzugsweise auf eine Temperatur von 40 - 60°C und der pH-Wert des Reaktionsgemisches durch Zugabe einer Base, beispielsweise NaOH, KOH u. dgl. auf etwa 8 - 11, vorzugsweise 9 - 10 eingestellt, und anschließend das Gemisch auf Raumtemperatur abgekühlt. Das Waschen des Reaktionsgemisches und das Fällen der Zielverbindung erfolgt analog dem oben angegebenen Vorgang.

Wird die 2-Chinolinylthioessigsäure mit Salzsäure als Hydrochlorid isoliert, kann die Freisetzung der Säure durch Lösen des Hydrochlorides beispielsweise in einer wässrigen Lösung von Natriumhydrogencarbonat oder Natriumcarbonat oder dgl. und erneute Fällung im sauren Bereich, beispielsweise bei einem pH-Wert von etwa 3 - 4 erfolgen.

Erfolgt die Umsetzung in saurem Medium, so wird die aufgeschmolzene Ausgangsverbindung 2-Chlorchinolin in einem wäßrigen Medium, beispielsweise destilliertem Wasser, aufgenommen und eine anorganische Säure, beispielsweise konzentrierte HCl, zugefügt. Anschließend wird Mercaptoessigsäure zudosiert und das Reaktionsgemisch erhitzt. Die Temperatur beträgt dabei 45°C bis zur Rückflußtemperatur der Reaktionsmischung. Nach Beendigung der Reaktion wird das Reaktionsgemisch auf Raumtemperatur bis 0°C abgekühlt und gegebenenfalls mit einem inerten organischen wassermischbaren Lösungsmittel, beispielsweise Aceton verdünnt.

Anschließend kann der pH-Wert der Lösung mittels einer anorganischen Base auf etwa 8 - 11, vorzugsweise 9 - 10 eingestellt werden; die Isolierung der freien Säure erfolgt wie oben angegeben.

Die Reaktionszeit der angegebenen Verfahrensschritte ist abhängig von der Reaktionstemperatur und beträgt etwa 10 min bis 24 h, vorzugsweise 30 min bis 12 h.
Der Fortgang der Reaktion wird in konventioneller Weise beispielsweise mittels Dünnschichtchromatographie verfolgt.

2- Chinolinylthioessigsäure stellt ein wertvolles Zwischenprodukt zur Herstellung von 2-Tribrommethylsulfonylchinolin dar, das ein sehr wirksamer Stabilisator in photothermographischem Material ist und eine ausgezeichnete Hautverträglichkeit aufweist.

Als Zwischenprodukt im in der EP - A 0 631 176 beschriebenen Verfahren kann allerdings auch das nach diesem Verfahren hergestellte Hydrochlorid der 2-Chinolinylthioessigsäure eingesetzt werden. Dazu wird im ersten Schritt das Natriumsalz der 2-Chinolinylthioessigsäure durch Zugabe von 2 Äquivalenten einer entsprechenden Base, beispielsweise Natriumhydroxid gebildet. Die weitere Reaktionsführung erfolgt wie in der EP - A 0 631 176 beschrieben.
Das Natriumsalz der 2-Chinolinylthioessigsäure wird mit Brom und Natriumhydroxid bei einer Temperatur von weniger als 30°C reagieren gelassen, der Niederschlag abgesaugt und getrocknet. Gegebenenfalls kann anschließend umkristallisiert werden.

### Beispiel 1

150,0 g (0,917 mol) 2-Chlorchinolin wurden bei 60 - 70°C aufgeschmolzen und anschließend 400 ml NaOH 10 %ig (ca. 1,2 Äquiv.) und 1,5 g (0,004 Äquiv.) Tetrabutylammoniumiodid zugegeben. Daraufhin wurde eine Lösung von 105,6 g (1,25 Äquiv.) Mercaptoessigsäure in 400 ml NaOH 10 %ig (ca. 1,2 Äquiv.) bei 70 - 80°C innerhalb von 30 min zudosiert. Das Reaktionsgemisch wurde 13 h unter Rückfluß gekocht, auf ca. 25°C abgekühlt und 3 x mit je 200 ml t-Butylmethylether gewaschen.
Die wässrige Phase wurde daraufhin mittels Salzsäure konz. auf einen pH-Wert < 1 bei max. 5°C angesäuert. Das ausgefallene Hydrochlorid wurde abgesaugt und 2 x mit 250 ml Wasser gewaschen. Der feuchte Filterrückstand wurde anschließend in 800 ml Wasser suspendiert und durch Zugabe von 160 g Natriumhydrogencarbonat gelöst.
2- Chinolinylthioessigsäure wurde durch Ansäuern auf pH 3 - 4 mittels Salzsäure konz. gefällt, abgesaugt und der Filterkuchen mit 2 x 200 ml Wasser gewaschen.
Nach dem Trocknen bei 50- 60°C im Vakuum wurden 158,9 g (79 % d.Th.) der Zielverbindung (Gehalt (HPLC): 99,5 % ) erhalten.
Schmp.: 90,5 - 91,9°C

### Beispiel 2

150,0 g (0,917 mol) 2-Chlorchinolin wurden vorgelegt und bei 60 - 70°C aufgeschmolzen. Anschließend wurden 530 ml NaOH 15 %ig (ca. 2,5 Äquiv.) und 1,5 g (0,004 Äquiv.) Tetrabutylammoniumiodid zugegeben und die Mischung auf Rückflußtemperatur (100 - 105°C) gebracht. Innerhalb von 5 min wurden dann 105,6 g (1,25 Äquiv.) Mercaptoessigsäure zudosiert und bei obiger Temperatur das Reaktionsgemisch 13,5 h unter Rückfluß gekocht.

Das Reaktionsgemisch wurde mit 250 ml Wasser verdünnt und auf ca. 25°C abgekühlt. Anschließend wurde das Reaktionsgemisch dreimal mit 200 ml t-Butylmethylether gewaschen.
Die wäßrige Phase wurde daraufhin mittels Salzsäure konz. auf einen pH-Wert < 1 bei max. 5°C angesäuert. Das ausgefallene Hydrochlorid wurde abgesaugt und der Filterkuchen 2 x mit 200 ml Wasser gewaschen.
Nach dem Trocknen bei 50 - 60°C im Vakuum wurden 193,4 g (83 % d.Th.) des Hydrochlorides der Zielverbindung (Gehalt (HPLC): 97,0 %) erhalten.

### Beispiel 3

250,0 g (1,528 mol) 2-Chlorchinolin wurden vorgelegt und bei 60 - 70°C aufgeschmolzen. Anschließend wurden 1340 ml NaOH 10 %ig (ca. 2,4 Äquiv.) und 2,5 g (0,004 Äquiv.) Tetrabutylammoniumiodid zugegeben. Innerhalb von 5 min wurden bei 70 - 80°C 176,0 g (1,25 Äquiv.) Mercaptoessigsäure zudosiert. Dann wurde das Reaktionsgemisch 13 h unter Rückfluß gekocht.
Nach dem Abkühlen auf ca. 25°C wurde das Reaktionsgemisch 3 x mit 300 ml t-Butylmethylether gewaschen. Die wässrige Phase wurde mit Salzsäure konz. bei max. 5°C auf pH 3 - 4 angesäuert.
Das Produkt wurde abgesaugt und 2 x mit 250 ml Wasser gewaschen.
Nach dem Trocknen bei 50 - 60°C im Vakuum wurden 316,3 g (94 % d.Th.) der Zielverbindung (Gehalt (HPLC): 99,3 %) erhalten.
Schmp.: 92,3 - 96,6°C

### Beispiel 4

25,0 g (0,153 mol) 2-Chlorchinolin wurden vorgelegt und bei 60 - 70°C aufgeschmolzen. Nach der Zugabe von 140 ml Wasser und 0,25 g (0,004 Äquiv.) Tetrabutylammoniumiodid wurden innerhalb von 5 min 17,6 g (1,25 Äquiv.) Mercaptoessigsäure zudosiert. Anschließend wurde das Reaktionsgemisch 1 h unter Rückfluß gekocht. Nach dem Abkühlen auf ca. 20°C wurde durch Zugabe von NaOH 50 %ig der pH-Wert auf 8 - 9 eingestellt.
Anschließend wurde bei max. 5°C auf pH 3 - 4 angesäuert, dann das Produkt abgesaugt und 2 x mit 50 ml Wasser gewaschen.

Nach dem Trocknen bei 50 - 60°C im Vakuum wurden 30,1 g (90 % d.Th.) der Zielverbindung (Gehalt (HPLC): 80,2 %) erhalten.
Schmp.: 79,8 - 81,5°C

### Beispiel 5

92,0 g (0,562 mol) 2-Chlorchinolin wurden vorgelegt und bei 60 - 70°C aufgeschmolzen. Nach der Zugabe von 350 ml Wasser wurden 64,8 g (1,25 Äquiv.) Mercaptoessigsäure innerhalb von 5 min zudosiert.
Das Reaktionsgemisch wurde 50 min unter Rückfluß gekocht und anschließend auf ca. 50°C abgekühlt.
Durch Zugabe von NaOH 50 %ig wurde der pH-Wert auf 8 - 9 eingestellt.
Das Reaktionsgemisch wurde auf 20 - 25°C abgekühlt und 3 x mit 150 ml Ethylacetat gewaschen.
Die wässrige Phase wurde bei max. 5°C auf pH 3 - 4 mit Salzsäure konz. angesäuert, das Produkt abgesaugt und 2 x mit 150 ml Wasser gewaschen.
Nach dem Trocknen bei 50 - 60°C im Vakuum wurden 105,2 g (85 % d.Th.) der Zielverbindung (Gehalt (HPLC): 92,3 %) erhalten.
Schmp.: 85,8 - 87,5°C

### Beispiel 6

200,0 g (1,222 mol) 2-Chlorchinolin wurden vorgelegt und bei 60 - 70°C aufgeschmolzen.
Nach der Zugabe von 750 ml Wasser wurden innerhalb von 20 min bei 80 - 85°C 124,3 g (1,10 Äquiv.) Mercaptoessigsäure zudosiert.
Das Reaktionsgemisch wurde 1h bei 80 - 85°C gerührt, auf ca. 40°C abgekühlt, der pH-Wert durch Zugabe von NaOH 32 %ig auf 9 - 10 eingestellt und auf 20 - 25°C abgekühlt.
Das Reaktionsgemisch wurde 3 x mit 250 ml Toluol gewaschen und dann mit 300 ml Wasser verdünnt. Die wässrige Phase wurde auf pH 3 - 4 bei max. 5°C mit Salzsäure konz. angesäuert, das Produkt abgesaugt und 3 x mit 250 ml Wasser gewaschen.

Nach dem Trocknen bei 50 - 60°C im Vakuum wurden 232,9 g (87 % d.Th.) der Zielverbindung (Gehalt (HPLC): 91,8%) erhalten.
Schmp.: 86,9 - 89,3°C

### Beispiel 7

100,0 g (0,611 mol) 2-Chlorchinolin wurden vorgelegt, bei 60 - 70°C aufgeschmolzen und 500 ml Wasser zugegeben.
Anschließend wurden innerhalb von 5 min bei 45 - 50°C 70,4 g (1,25 Äquiv.) Mercaptoessigsäure zudosiert, dann das Reaktionsgemisch 13 h bei 45 - 50°C gerührt, der pH-Wert auf 9 - 10 durch Zugabe von NaOH 32 %ig eingestellt, auf 20 - 25°C abgekühlt und 3 x mit 150 ml Toluol gewaschen.
Nach dem Verdünnen des Reaktionsgemisches mit 350 ml Wasser wurde die wässrige Phase auf pH 3 - 4 bei max. 5°C mit Salzsäure konz. angesäuert, das Produkt abgesaugt und 3 x mit 250 ml Wasser gewaschen.
Nach dem Trocknen bei 50- 60°C im Vakuum wurden 113,2 g (85% d.Th.) der Zielverbindung (Gehalt (HPLC): 98,2 %) erhalten.
Schmp.: 89,5 - 91,0°C

### Beispiel 8

100,0 g (0,611 mol) 2-Chlorchinolin wurden vorgelegt, bei 60 - 70°C aufgeschmolzen und 500 ml Wasser zugegeben. Nach dem Zudosieren von 70,4 g (1,25 Äquiv.) Mercaptoessigsäure innerhalb von 5 min bei 95°C wurde das Reaktionsgemisch 20 min unter Rückfluß bei 100 - 105°C gekocht, anschließend auf 40 - 45°C abgekühlt, der pH-Wert durch Zugabe von NaOH 32 %ig auf 9 - 10 eingestellt, auf 20 - 25°C abgekühlt und das Reaktionsgemisch 3 x mit 150 ml Toluol gewaschen.
Anschließend wurde das Reaktionsgemisch mit 350 ml Wasser verdünnt, die wässrige Phase bei max. 5°C mit Salzsäure konz. auf pH 3 - 4 angesäuert, das Produkt abgesaugt und 3 x mit 250 ml Wasser gewaschen.
Nach dem Trocknen bei 50 - 60°C im Vakuum wurden 106,4 g (79 % d.Th.) der Zielverbindung (Gehalt (HPLC): 91,7 %) erhalten.
Schmp.: 86,7 - 89,3°C

### Beispiel 9

100,0 g (0,611 mol) 2-Chlorchinolin wurden vorgelegt, bei 60 - 70°C aufgeschmolzen und 500 ml Wasser und 51 ml (1,0 Äquiv.) Salzsäure konz. zugegeben.
Anschließend wurden 70,4 g (1,25 Äquiv.) Mercaptoessigsäure innerhalb von 5 min unter Rückfluß zudosiert und das Reaktionsgemisch 20 min unter Rückfluß gekocht.
Nach dem Abkühlen auf 40 - 45°C wurde der pH-Wert durch Zugabe von NaOH 32 %ig auf 9 - 10 eingestellt, das Reaktionsgemisch auf 20 - 25°C abgekühlt, 3 x mit 150 ml Toluol gewaschen und mit 350 ml Wasser verdünnt.
Die wässrige Phase wurde auf pH 3 - 4 bei max. 5°C mit Salzsäure konz. angesäuert, das Produkt abgesaugt und 3 x mit 250 ml Wasser gewaschen.
Nach dem Trocknen bei 50 - 60°C im Vakuum wurden 111,8 g (83 % d.Th.) der Zielverbindung (Gehalt (HPLC): 84,5 %) erhalten.
Schmp.: 85,5 - 87,3°C

### Beispiel 10

100,0 g (0,611 mol) 2-Chlorchinolin wurden vorgelegt, bei 60 - 70°C aufgeschmolzen und 500 ml Wasser und 101 ml (2,0 Äquiv.) Salzsäure konz. zugegeben. Dann wurden 70,4 g (1,25 Äquiv.) Mercaptoessigsäure innerhalb von 5 min. unter Rückfluß zudosiert, das Reaktionsgemisch 30 min unter Rückfluß gekocht, auf 0 - 5°C abgekühlt, mit 100 ml Aceton verdünnt und das Produkt-Hydrochlorid abgesaugt. Es wurde 2 x mit 100 ml Aceton und 2 x mit 100 ml Wasser nachgewaschen. Der feuchte Filterrückstand wurde in 500 ml Wasser suspendiert, der pH-Wert auf 9 - 10 durch Zugabe von NaOH 32 %ig eingestellt und auf 20 - 25°C abgekühlt.
Das Reaktionsgemisch wurde mit 3 x 200 ml Toluol gewaschen, die wässrige Phase auf pH 3 - 4 bei max. 5°C mit Salzsäure konz. angesäuert, das Produkt abgesaugt und 3 x mit 250 ml Wasser gewaschen.

Nach dem Trocknen bei 50 - 60°C im Vakuum wurden 106,0 g (79 % d.Th.) der Zielverbindung (Gehalt (HPLC): 80,9 %) erhalten.
Schmp.: 79,3 - 80,3°C

### Beispiel 11

100,0 g (0,611 mol) 2-Chlorchinolin wurden vorgelegt, bei 60 - 70°C aufgeschmolzen und 400 ml Wasser und 101 ml (2,0 Äquiv.) Salzsäure konz. zugegeben. Anschließend wurden 70,4 g (1,25 Äquiv.) Mercaptoessigsäure innerhalb von 5 min. bei 70°C zudosiert, das Reaktionsgemisch 6 h bei 65 - 70°C gerührt, dann auf 0 - 5°C abgekühlt und 30 min bei 0 - 5°C gerührt.
Das Produkt-Hydrochlorid wurde abgesaugt und 2 x mit 100 ml Wasser (0 - 5°C) gewaschen.
Nach dem Trocknen bei 50 - 60°C im Vakuum wurden 142,6 g (91 % d.Th.) des Hydrochlorides der Zielverbindung (Gehalt (HPLC): 83,5 %) erhalten.
Schmp.: 206,6 - 208,6°C

## Patentansprüche

1. Verfahren zur Herstellung von 2-Chinolinylthioessigsäure der Formel I 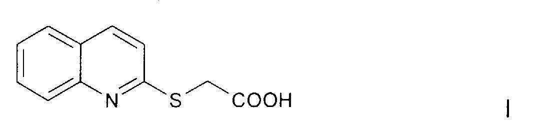 und deren Salze, dadurch gekennzeichnet, dass man 2-Chlorchinolin der Formel II 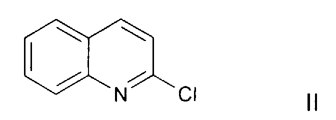 in wässriger Lösung mit Mercaptoessigsäure umsetzt und gegebenenfalls das erhaltene Salz in die freie Säure oder die freie Säure und ein Salz überführt.

2. Verfahren nach Anspruch 1, dadurch gekennzeichnet, dass die Umsetzung in basischem wässrigem Medium erfolgt.

3. Verfahren nach einem der Ansprüche 1 oder 2, dadurch gekennzeichnet, dass die Umsetzung in Gegenwart von Natriumhydroxid bei einem pH-Wert von 9 bis 10 erfolgt.

4. Verfahren nach einem der Ansprüche 1 bis 3, dadurch gekennzeichnet, dass die Einstellung des pH-Wertes auf einen Wert von 9 - 10 erst nach Umsetzung von 2-Chlorchinolin mit Mercaptoessigsäure erfolgt.

5. Verfahren nach einem der Ansprüche 1 bis 4, dadurch gekennzeichnet, dass die Umsetzung in Gegenwart von Tetrabutylammoniumiodid erfolgt.

6. Verfahren nach Anspruch 1, dadurch gekennzeichnet, dass die Umsetzung in saurem wässrigem Medium erfolgt.

7. Verfahren nach einem der Ansprüche 1 oder 6, dadurch gekennzeichnet, dass die Umsetzung in Gegenwart von Salzsäure erfolgt.

8. Verfahren nach einem der Ansprüche 1 bis 7, dadurch gekennzeichnet, dass die Umsetzung bei Temperaturen von 35°C bis zur Rückflußtemperatur der Reaktionsmischung durchgeführt wird.

9. Verfahren nach einem der Ansprüche 1 bis 8, dadurch gekennzeichnet, dass das Hydrochlorid der 2-Chinolinylthioessigsäure isoliert wird.

10. Verfahren nach einem der Ansprüche 1 bis 9, dadurch gekennzeichnet, dass die freie 2-Chinolinylthioessigsäure isoliert wird.
